# EUROPEAN PATENT SPECIFICATION

(11) **EP 1 814 976 B1**
(45) Date of publication and mention of the grant of the patent: **07.01.2015**
(21) Application number: 05851637.8
(22) Date of filing: 16.11.2005
(51) Int. Cl.: C12M 3/00

(54) **PEPTIDOMIMETIC POLYMERS FOR ANTIFOULING SURFACES**
PEPTIDOMIMETISCHE POLYMERE FÜR ANTIFOULING-OBERFLÄCHEN
POLYMERES PEPTIDOMIMETIQUES POUR SURFACES ANTISALISSURE

(30) Priority: 16.11.2004 US 628359 P
(43) Date of publication of application: 08.08.2007
(73) Proprietor: Northwestern University, Evanston, IL 60208 (US)
(72) Inventor: MESSERSMITH, Phillip, B., Clarendon Hills, IL 60514 (US); BARRON, Annelise, E., Evanston, IL 60626-1159 (US); STATZ, Andrea, Evanston, IL 60201 (US); MEAGHER, Robert, Evanston, IL 60201 (US)
(74) Representative: Venner, Julia Ann
(86) International application number: PCT/US2005/041280
(87) International publication number: WO 2006/055531

(56) References cited:
- US-A- 5 410 023
- US-A1- 2003 087 338
- US-B1- 6 306 993
- STATZ A R ET AL: "New peptidomimetic polymers for antifouling surfaces", JOURNAL OF THE AMERICAN CHEMICAL SOCIETY, AMERICAN CHEMICAL SOCIETY, WASHINGTON, DC; US, vol. 127, no. 22, 1 January 2005 (2005-01-01), pages 7972-7973, XP002541457, ISSN: 0002-7863, DOI: DOI:10.1021/JA0522534 [retrieved on 2005-05-13]
- DALSIN ET AL: "Bioinspired antifouling polymers", MATERIALS TODAY, ELSEVIER SCIENCE, KIDLINGTON, GB, vol. 8, no. 9, 1 September 2005 (2005-09-01), pages 38-46, XP005024450, ISSN: 1369-7021, DOI: DOI:10.1016/S1369-7021(05)71079-8
- BRANCH D W ET AL: "Long-term stability of grafted polyethylene glycol surfaces for use with microstamped substrates in neuronal cell culture", BIOMATERIALS, ELSEVIER SCIENCE PUBLISHERS BV., BARKING, GB, vol. 22, no. 10, 1 May 2001 (2001-05-01), pages 1035-1047, XP004232536, ISSN: 0142-9612, DOI: DOI:10.1016/S0142-9612(00)00343-4
- MURPHY J E ET AL: "A combinatorial approach to the discovery of efficient cationic peptoid reagents for gene delivery", PROCEEDINGS OF THE NATIONAL ACADEMY OF SCIENCES, NATIONAL ACADEMY OF SCIENCES, WASHINGTON, DC; US, vol. 95, no. 4, 17 February 1998 (1998-02-17), pages 1517-1522, XP002235261, ISSN: 0027-8424, DOI: DOI:10.1073/PNAS.95.4.1517
- VREELAND WYATT N ET AL: "Multiplexed, high-throughput genotyping by single-base extension and end-labeled free-solution electrophoresis.", ANALYTICAL CHEMISTRY 1 SEP 2002 LNKD- PUBMED:12236339, vol. 74, no. 17, 1 September 2002 (2002-09-01), pages 4328-4333, XP002653787, ISSN: 0003-2700

## Description

### BACKGROUND OF THE INVENTION

Protein, cell, and bacterial fouling of surfaces occur spontaneously upon exposure of medical implants and diagnostic devices to physiologic fluids and tissues. In many cases biofouling is an adverse event that can impair function or even cause catastrophic failure of medical devices. Examples of problematic biofouling include occlusion of cardiovascular implants by thrombus, protein accumulation onto biosensor surfaces, and bacterial colonization of indwelling catheters. Complications arising from fouling of medical implants and devices significantly increase the cost of healthcare delivery and can lead to reduction of implant performance, implant failure, and patient infections.

Strategies in the art for inhibiting biofouling are directed to grafting antifouling polymers or self-assembled monolayers (SAMs) onto surface. Technical issues critical to the longevity and antifouling performance of such organic coatings include the nature of the chemical bond used for anchoring such coatings onto surfaces, as well as the chemical characteristics of the polymer/SAM. Common anchoring chemistries include thiol- and silane-containing molecules on metals and metal oxides, respectively, electrostatic interactions between polyelectrolytes and charged surfaces, and numerous strategies that take advantage of reactive organic functional groups on surfaces and molecules in solution or the vapor phase. While oligoethylene glycol terminated SAMs have shown excellent antifouling properties, their stability under *in-vivo* conditions may be limited in certain applications. A variety of polymers have been investigated as antifouling coatings, including poly(ethylene glycol) (PEG), poly(methoxyethyl acrylate) (PMEA), poly(phosphorylcholine methacrylate), and glycomimetic polymers. Each of these polymers has met with some success in *in-vitro* and *in-vivo* antifouling tests. However, none have yet proven to be ideal for long-term prevention of protein, cell, and bacterial fouling of surfaces.

Branch et al, Biomaterials (2001) Vol 22, No. 10, p. 1035-1047 discloses PEG-based antifouling materials, which are stable for about one month.

### BRIEF SUMMARY OF THE INVENTION

In one aspect, the present invention comprises a peptidomimetic polymer comprising a coupled polypeptide moiety and a polypeptoid moiety, the polypeptide moiety consisting of a 5-mer peptide mimic of Mefp-5 comprising alternating residues of L-3, 4-dihydroxyphenylalanine (DOPA) and lysine, wherein the polypeptoid moiety comprises a poly-N-substituted glycine oligomer, wherein the N- substituent is methoxy ethyl.

A general preparative route for the coupled moieties of this invention, i.e., the peptidomimetic composition or coating, is shown in Scheme 1, as follows: 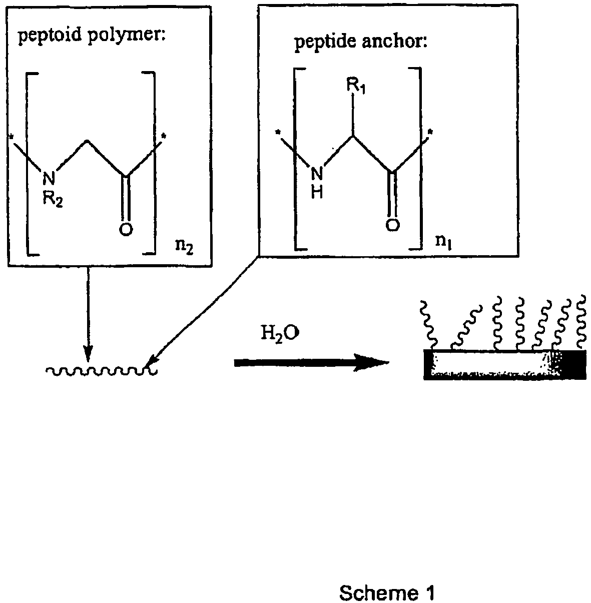 Scheme 1 shows the general synthetic route for peptidomimetic compositions of the invention as well as a schematic showing of a peptidomimetic coated surface (greatly magnified) of this invention.

The general design of such chimeric polymers of this invention can be described in a preferred aspect as a functional peptide domain component for robust adsorption deposition, adhesive, absorptive, or other interaction with surfaces, coupled or conjugated to an N-substituted glycine peptoid polymer component that is resistant to protein and cell fouling. The synthetic approach is versatile and allows virtually unlimited variation of composition. Modification of a variety of surfaces can be accomplished with a simple aqueous solution-based adsorption or deposition strategy. In certain embodiments, a peptidomimetic polymer of this invention can be adsorbed to, or deposited on, a metal oxide surface (e.g., titanium oxide), which in turn exhibits significantly reduced serum protein adsorption, and inhibited cell fouling for weeks, months, to several months (or longer) under *in-vitro* conditions.

The preferred class of anchoring peptide domain or moiety was chosen to mimic the adhesive proteins used by marine mussels to attach to underwater surfaces. Mussels are known for their ability to adhere strongly to a variety of wet surfaces, and for this purpose secrete liquid "glues" containing mussel adhesive proteins (MAPs), which rapidly harden to form a solid adhesive plaque.
The adhesive or anchor moiety comprises dihydroxyphenyl derivatives (DHPD) including, di-(DHPD) wherein the second DHPD is 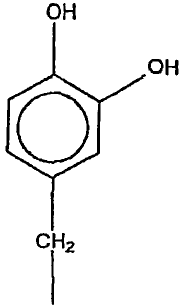 The DHPD is L, 3, 4 dihydroxyphenyl alanine (DOPA) which is more completely described below.

### BRIEF DESCRIPTION OF THE FIGURES

Figure 1 illustrates the molecular structure of a preferred peptidomimetic polymer composition of this invention.
Figure 2 shows the low mass region of the positive ion ToF-SIMS spectra of Ti modified with a peptidomimetic polymer coating.
Figure 3 shows mid-range mass region of the negative ion ToF-SIMS spectra of Ti modified with peptidomimetic polymer.
Figure 4 is a high resolution C(1s) XPS spectra of unmodified and polypeptoid modified TiO₂ substrates.
Figure 5 is a high-resolution N(1s) XPS spectra of polypeptoid modified TiO₂ substrate.
Figure 6 is a mass plot of scrum protein adsorption on peptoid modified Ti waveguide as measured by OWLS.
Figure 7 is a total projected cell area of 3'1'3 fibroblasts on TiO₂ and peptoid modified TiO₂. Cells were reseeded twice weekly throughout the duration of the experiment.

### DETAILED DESCRIPTION OF THE INVENTION

The adhesive properties of the anchoring protein moieties of this invention are believed to be due to the presence of L-3,4- dihydroxyphenylalanine (DOPA), an amino acid that is formed by post-translational modification of tyrosine. Of the several blue mussel (*Mytilus edulis*) adhesive pad proteins identified in the art, Mefp-3 and Mefp-5 are of special interest because these proteins have high DOPA content and are structurally located closest to the interface between the adhesive pad and substrate. See, Waite, J.H. and X. Qin, Polyphosphoprotein from the Adhesive Pads of Mytilus edulis. Biochemistry, 2001. 40: p. 2887-2893. Papov, V.V., et al., Hydroxyarginine-containing Polyphenolic Proteins in the Adhesive Plaques of the Marine Mussel Mytilus edulis. Journal of Biological Chemistry, 1995. 270(34): p. 20183-20192. At approximately 27%, Mefp-5 has the highest DOPA content of any isolated MAP. Furthermore, over 75% of the DOPA residues in Mefp-5 are immediately adjacent to lysine (Lys) residues.

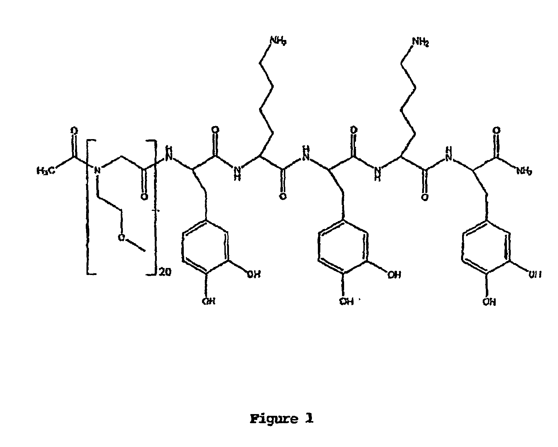

Accordingly, the invention employs a 5-mer peptide mimic of Mefp-5 comprising alternating DOPA and Lys residues (Figure 1) as an anchor for polymer immobilization. Without restriction to any one theory or mode of operation, the catechol side chains of the DOPA residues are hypothesized to form charge transfer complexes to metal oxide surfaces, whereas the cationic nature of the Lys residues should provide electrostatic attraction to a negatively charged oxide surface. Other DOPA-containing peptide anchor components useful in conjunction with the present inventive polymers will be understood by those skilled in the art made aware of this invention, such components including but not limited to those described in co-pending application serial no. 10/199,960 (U.S. Patent Application Publication 2003-0087338 published May 8, 2003 particularly paragraphs [0089] through [0092]) and application serial no. 10/699,584 (U.S. Patent Application Publication 2004/026595 published December 30, 2004), filed July 19, 2002 and October 31, 2004, respectively.

An antifouling portion of the polymer can comprise a poly-N-substituted glycine oligomer (peptoid) of variable length. Peptoids are non-natural mimics of peptides that have a protein-like backbone, with side chain derivatization at the amide nitrogen instead of the alpha-carbon Formula II. A wide range of N-substituents, corresponding N-substituted glycine residues and related peptoid components will be understood by those skilled in the art made aware of this invention, such residues and peptoid components as can be prepared as described below, in the referenced prior art, U.S. Pat. No. 6,887,845, and/or in co-pending application serial no. 11/120,071 filed May 2, 2005.

In certain embodiments, the peptoid component can comprise an N-substituted methoxyethyl side chain, according to current understanding in the art of functional groups that provide fouling resistance to surfaces. In an extensive study of protein adsorption onto functionalized SAMs, certain functional group characteristics were identified that render surfaces resistant to protein adsorption. They include hydrophilicity, hydrogen-bond acceptors but not hydrogen-bond donors, and electrical charge neutrality. See, Ostuni, E., et al., A Survey of Structure-Property Relationships of Surfaces that Resist the Adsorption of Protein. Langmuir, 2001. 17: p. 5605-5620 which is incorporated by reference herein. Like PEG and PMEA components, the methoxyethyl side chain of such polypeptoid exhibits all four of these characteristics, including hydrophilicity, hydrogen bond acceptors, no hydrogen bond donors, and no charge. N-substituent identity is limited only by functional effect, meeting one or more of the aforementioned characteristics and/or otherwise demonstrating antifouling properties upon incorporation or one or more such moletics into a corresponding peptoid polymer component. With respect to the chemical properties of the peptoid backbone, side chain substitution from the nitrogen (instead of the alpha carbon peptides) eliminates the amide hydrogen, removes the capacity for hydrogen bond donation, and significantly decreases incidence of protease degradation.

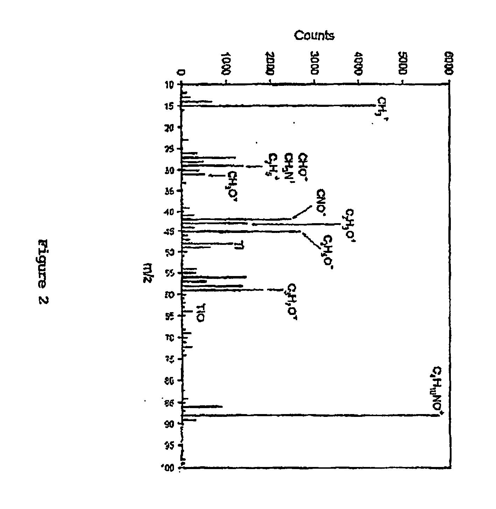

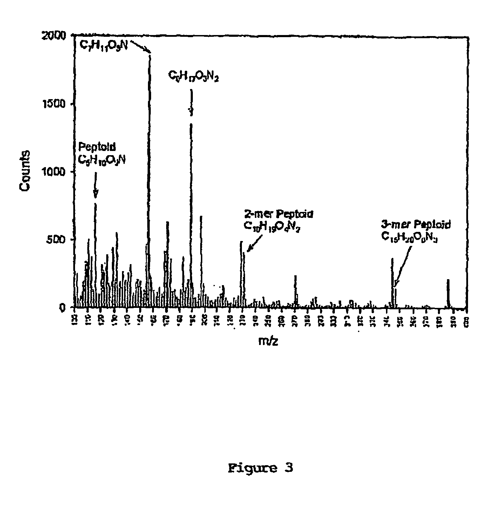

The chimeric peptide-peptoid molecule shown in Scheme 1 was synthesized on solid phase resin by first synthesizing the adhesive peptide anchor with standard Fmoc strategy followed by synthesis of a 20-mer N-methoxyethyl glycine peptoid using a known submonomer protocol. See, Zuckerman, R.N., et al., Efficient Method For the Preparation of Peptoids [Oligo(n-Substituted Blycines)] By Submonomer Solid Phase Synthesis. Journal of the American Chemical Society, 1992. 114(26): p. 10646-10647. The amine terminus was acetylated, cleaved from the resin, purified by RP-HPLC and analyzed by mass spectrometry. Silicon wafers coated with 20nm of electron beam evaporated Ti were modified by adsorption of the peptidomimetic polymer from an aqueous solution. Unmodified and modified Ti surfaces were analyzed by time-of-flight secondary ion mass spectrometry (ToF-SIMS) and X-ray photoelectron spectroscopy (XPS). The positive ion ToF-SIMS spectrum of unmodified Ti (not shown) exhibited typical low intensity hydrocarbon contamination peaks (CₙH₂ₙ₋₁⁺) and (CₙH₂ₙ₋₁⁺), a Ti peak at m/z = 47.95 and a TiO¹ peak at *m*/*z* = 63.95. The positive ion ToF-SIMS spectrum of peptoid modified Ti revealed numerous fragments representing the presence of adsorbed peptoid polymer (Figure 2). Apparent fragmentation of the methoxyethyl side chain gave rise to peaks for CH₃¹ (*m*/*z* = 15) C₂H₅O⁺ (*m*/*z =* 45.06) and C₃H₇O⁺ (*m*/*z* = 59.07) fragments, as well as Lys-derived fragments such as C₂NH₄⁺ (*m*/*z* = 42.0). The negative ion spectrum contained peaks for 1-, 2- and 3-mer peptoid fragments as well as other large fragments of the peptoid portion of the polymer (Figure 3).

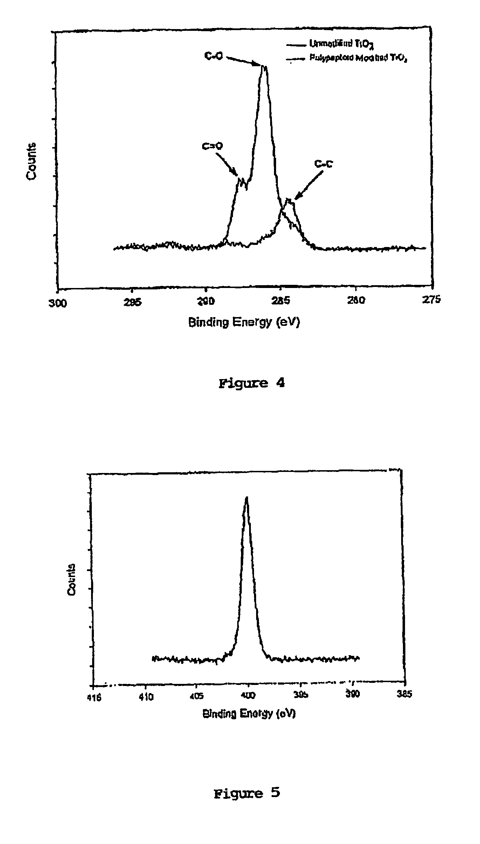

XPS analysis of the surfaces modified with the peptidomimetic polymer reveals further evidence of peptidomimetic polymer adsorption onto the Ti surface. XPS spectra showed an increase in the ether (C-O) peak at 286.0 cV when compared to control Ti surfaces (Figure 4). A peak at 284.6 eV is due to the aliphatic and aromatic carbons in the methoxyethyl side chain and the DOPA anchoring group, as well as hydrocarbon contamination. The carbonyl groups of the peptidometic polymer backbone are represented by a peak at 287.5 eV. Furthermore, a strong N(1s) peak was present at 399.7 eV in the spectra of the polypeptoid modified surfaces that was not observed in the unmodified Ti surfaces (Figure 5). While certain embodiments of this invention are demonstrated in conjunction with a titanium oxide substrate, it will be understood by those skilled in the art that various other materials, including but not limited to other metal oxides, can be employed with composites and/or compositions comprising any one or a plurality of the peptidomimetic polymers of this invention. Other such materials include those recognized in the art for implementation of the medical and non-medical applications mentioned herein.

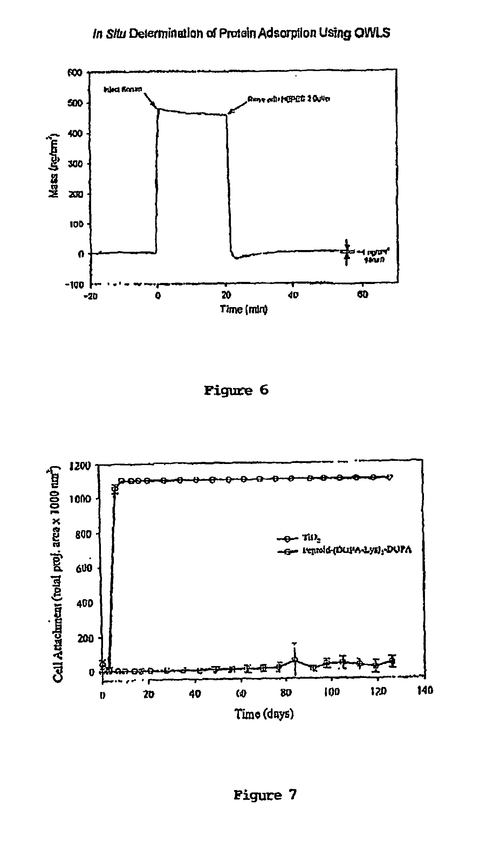

Optical waveguide lightmode spectroscopy (OWLS) experiments revealed that polypeptoid modification of titanium surfaces resulted in a substantial reduction in protein adsorption (Figure 6). Exposure of unmodified Ti waveguides to whole human serum for 20 minutes resulted in an adsorbed protein layer with a mass between 150 and 230 ng/cm² after rinsing. However, surprisingly and unexpectedly, serum protein adsorption onto representative peptoid modified substrates of this invention under identical conditions was reduced to approximately 4ng/cm². This amount of protein adsorption is similar to that adsorbed onto DOPA-anchored PEG coatings and to oligoethylene glycol terminated SAMs, demonstrating the excellent protein resistance of peptidomimetic polymer compositions of the invention.

Finally, the ability of polypeptoid modified surfaces to resist cell attachment over a long period of time was determined by culturing 3T3 fibroblast cells on unmodified and modified titanium surfaces in the presence of serum. Fresh cells were seeded twice weekly onto the titanium surfaces for several months, the cell attachment at various time points assayed by fluorescence microscopy and image analysis. Although fibroblasts readily attached to unmodified titanium surfaces and were nearly confluent after several days (Figure 7), the peptoid modified surfaces exhibited low levels of cell attachment throughout the experiment. Since cell attachment to surfaces is typically mediated by adsorbed protein, the results infer that serum protein adsorption remained low throughout the course of the *in vitro* experiment.

It is interesting to note that fouling resistance persisted for many days in the presence of serum, which typically contains protease enzymes that would be expected gradually to degrade peptide bonds of the polymer backbone. In this respect as well, the design of the peptoid is beneficial in that the placement of the side chain on the amide nitrogen leads to an essentially protease-resistant backbone. Although the adhesive peptide anchor of the molecule may be susceptible to protease degradation, at high polymer density on the surface the peptide anchors are likely to be buried beneath or protected by the peptoid chains and therefore essentially inaccessible to serum proteases.

As illustrated, above, a new de novo designed peptidomimetic polymers of this invention were synthesized and determined to have excellent and long-lasting antifouling properties when immobilized onto a metal oxide surface. Such chimeric compounds can comprise a mussel adhesive protein mimetic peptide for robust water-resistant anchorage onto substrates, coupled to an oligometric N-substituted glycine peptoid with a side chain (e.g., methoxyethyl) designed for resistance to protein and cell fouling. The modular, solid phase approach known in the art used to synthesize these peptidomimetic polymers offers precise control of molecular weight at high yields, and with virtually unlimited versatility in functionality obtained through variation of N-substituted side chain composition in the form of both natural and non-natural side chains. See, Zuckerman, R.N., et al., Efficient Method For the Preparation of Peptoids [Oligo(N-Substituted Glycines)] By Submonomer Solid Phase Synthesis. Journal of the American Chemical Society, 1992. 114(26): p. 10646-10647. Kirshenbaum, K., et al., Sequence-specific polypeptoids: A diverse family of heteropolymers with stable secondary structure. PNAS, 1998. 95(8): p. 4303-4308. The synthetic diversity available through such peptidomimetic polymers can be used to better understand the fundamental relationship between chemical composition of polymers and protein/cell resistance. Enhanced understanding of these relationships may, in turn, lead to improved antifouling strategies for medical and nonmedical applications.

### EXAMPLES

### Materials

Bromoacetic acid (BAA) and methoxyethylamine were purchased from Aldrich (Milwaukee, WI).

### Polymer Synthesis

A peptidomimetic polymer composition of the invention was synthesized on 0.25 mmol Fmoc-Rink amide resin (Nova Biochem, San Diego, CA) using an ABI 433A (Applied Biosystems, Foster City, CA) automated peptide synthesizer. Conventional Fmoc strategy of solid phase peptide synthesis with Fmoc-Lys-(N-Boc) and Fmoc-DOPA(acetonid) amino acids (Nova Biochem, San Diego, CA) was used to synthesize the *C-terminal* DOPA-Lys-DOPA-Lys-DOPA peptide anchor, after which the polypeptoid portion was synthesized using submonomer protocol described previously. *See Zuckerman, supra.* Bromoacetylation of the N-terminal amino was accomplished by vortexing 4.15 mL of 1.2M bromoacetic acid in DMF and 1 mL of diisopropylcarbodiimide (DIC) (Aldrich, Milwaukee, WI) with the resin for 60 min. After rinsing 4 times with 7mL of DMF, the resin was vortexed for 60 min. with 4 mL of 1M methoxyethylamine (Aldrich, Milwaukee, WI) in N-methylpyrrolidone (NMP) (Applied Biosystems, Foster City, CA) to introduce the side chain moiety. The liquid was then drained and the resin washed with 7mL of DMF. These two reaction cycles were repeated until the desired number of peptoid monomers was obtained.

Following completion of the synthesis, the N-terminus of the peptidomimetic polymer was acetylated with acetic anhydride (Applied Biosystems, Foster City, CA). Cleavage of the peptidomimetic polymer from the resin deprotection of the amino acid side chains was accomplished by treating the resin with 95% (v/v) trifluoroacetic acid (Acres Organics, Belgium) with 2.5% H₂O and 2.5% triisopropylsilane (Aldrich, Milwaukee, WI). The cleaved peptidomimetic polymer was isolated by filtration and rinsed several times with acetonitrile and water. The crude product was analyzed by reversed-phase HPLC using a Vydac C18 column and ESI-MS for purity and composition. Purification was performed by preparative HPLC, and purified fractions were frozen at -85°C and lyophilized.

### Substrate Preparation

Silicon wafers were coated with 20 nm of electron beam evaporated Ti and then cut into 8mm by 8mm pieces. The substrates were cleaned ultrasonically for ten minutes in 2-propanol, dried under N₂ and then exposed to O₂ plasma (Harrick Scientific Ossining, USA) at ≤| 50 Torr and 100W for three minutes to produce a clean titanium oxide surface. OWLS waveguides were purchased from Microvacuum Ltd. (Budapest, Hungary), consisting of a SiO₂ substrate coated with Si_{6.25}Ti_{6.75}O₂ and a final 10 nm thick coating of TiO₂ produced by a sol-gel process. Voros, J., et al., Optical grating coupler biosensors. Biomaterials, 2002. 23: p. 3699-3710. Sensors were cleaned following the same procedure as Ti substrates.

### Substrate Modification

Clean substrates and sensors were immersed in 1mg/ml peptidomimetic polymer in saturated NaCl buffered with 0.1M N-morpholinopropanesulfonic acid (MOPS) at 60°C for 24 hours to form a uniform monolayer. After modification, substrates were exhaustively rinsed with ultrapure H₂O and dried in a stream of filtered N₂.

### Surface Characterization

Survey and high resolution XPS spectra were collected on an Omicron FSCALAB (Omicron, Taunusstein, Germany) configured with a monochromated Al Kα (1486.8 eV) 300-W X-ray source, 1.5 nm circular spot size, a flood gun counter charging effects, and an ultrahigh vacuum (<10⁻⁸ Torr). The takeoff angle, defined as the angle between the substrate normal and the detector, was fixed at 45°. Substrates were mounted on standard sample studs by means for double-sided adhesive tape. All binding energies were calibrated using the C(1s) carbon peak (284.6 eV). Analysis included a broad survey scan (50.0 eV pass energy) and a 10-min. high-resolution scan (22.0 eV pass energy) at 270-300 eV for C(1s) and, comparably, for N(ls).

Secondary ion spectra were recorded on a TRIFT III time-of-flight secondary ion mass spectrometer (Physical Electronics, Eden Prairie, MN) in the mass range 0-2000 *m*/*z*. A Ga -source was used at a beam energy of 15 keV with a 100 µm raster size. Positive and negative spectra were collected and calibrated with a set of low mass ions using the PHI software Cadence.

### Cell Culture

3T3-Swiss albino fibroblasts (ATCC, Manassas, VA) were maintained at 37°C and 5% CO₂ in Dulbecco's modified Eagle's medium (DMEM, Cellgro, Herndon, VA) containing 10% fetal bovine serum (FBS) and 100 µm/ml of penicillin and 100 U/ml of streptomycin. Immediately before use, fibroblasts of passage 12-16 were harvested using 0.25% trypsin-EDTA, resuspended in DMEM with 10% FBS and counted using a hemacytometer.

### Quantification of Cell Adhesion

Modified and unmodified TiO₂ substrates were pretreated in a 12-well TCPS plate with 1.0 ml of DMEM containing FBS for 30 minutes at 37°C and 5% CO₂. Fibroblasts were seeded onto the test substrates at a density of 2.9 x 10³ cell/cm². For short-term studies, the substrates were maintained in DMEM with 10% FBS at 37°C and 5% CO₂ for 4 hours, after which adherent cells were fixed in 3.7% paraformaldehyde for 5 minutes and stained with 5 M 1,1'-dioctadecyl-3,3,3',3' tetramethylindocarbocyanine perchlorate (Dil; Molecular Probes, Eugene, OR) for inflorescent microscope counting. For long-term adhesion experiments substrates were reseeded with 3T3 fibroblasts at a density of 2.9 x 10³ cells/cm² twice per week. The medium was aspirated from each well to remove any non-adherent cells and PBS was used to rinse the substrates and wells. Fibroblasts were stained with 2.5 µM calcein-AM (Molecular Probes) in complete PBS for 1 hour at 37°C twice per week initially and then once per week after 2 weeks.

Quantitative cell attachment data was obtained by acquiring nine images from random locations on each substrate using an Olympus BX-40 (λ_{Ex} = 549nm, λ_{Em} = 565nm) and a Coolsnap CCD camera (Roper Scientific, Trenton, NJ). The experiments were performed in triplicate for statistical purposes, resulting in a total of 27 images per time point for each substrate. The resulting images were quantified using thresholding in Metamorph (Universal Imaging, Downington, PA).

### Protein Adsorption

For *in situ* protein adsorption experiments, TiO₂ coated waveguides were modified *ex situ* with polypeptoid. The waveguides were inserted in the OWLS flow-through cell and equilibrated by exposing to HEPES-2 buffer (10 mM HEPES, 150 mM NaCl, pH 7.4) for at least 6 hours to allow for complete exchange for ions at the TiO₂ surface. The measurement head was mounted in the sample chamber and heated to 37°C; the signal was recorded to ensure a stable baseline and thus adequate equilibrium time. Whole human serum (Control Serum N, Roche Diagnostics, Switzerland, reconstituted in ultrapure water) was injected into the flow-through cell. The waveguide was exposed to serum for 40 minutes and subsequently rinsed with HEPES-2 buffer for another.

The refractive index of solutions was measured in a refractometer (J157 Automatic Refractometer, Rudolph Research( under identical experimental conditions. A refractive index value of 1.33119 was used for the HEPES-2 buffer and a standard value of 0.182 cm³/g was used for the protein-adsorption calculations. The residual increase in signal intensity versus baseline measured by OWLS can be directly correlated to adsorbed mass of protein.

One skilled in this art will be prompted to think of many applications for its present invention in light of this disclosure. The compounds, compositions coatings, and/or composites of this invention can be applied, without limitation, to:
1) Medical Diagnostics and Therapies, including but not limited to
   (a) Preparation of Nonfouling Surfaces for
      - Biosensors
      - Cardiovascular implants
      - Catheters
      - Lubricious coatings on catheters, needles, and other percutaneous devices
      - Medical tubing (dialysis)
      - Implantable electronic devices (MEMS)
      - Corrosion resistant coatings on medical grade metal alloys (surface adsorbed catechols are unknown to enhance corrosion resistance of metals); and
   (b) Stabilization of Particles of Diagnostics ad Therapy, such as
      - Stabilization of proteins, peptides and other therapeutics under in-vivo conditions
      - Nanoparticle-based ex-vivo diagnostics (gold or quantum dot based technologies)
      - Nanoparticle-based in-vivo diagnostics
         ∘ Paramagnetic nanoparticle contrast agents for MRI
         ∘ Nanoparticles for optical imaging
      - Nanoparticle-Based Therapies
         ∘ Superparamagnetic magnetite nanoparticles for hyperthermia, and
2) Nonmedical Applications, including but not limited to
   - Corrosion resistant coatings (surface adsorbed catechols and polyphenols are known to enhance corrosion resistance of metals, and polyphenol polymers are currently used as corrosion resistant coatings)
   - Antifouling coatings on consumer goods (sunglasses, etc.)
   - Antifouling coatings on electronic devices (MEMS, etc.)
   - Antifouling/anti-icing coatings on aircraft
   - Stabilization of quantum dot suspensions
   - Stabilization of magnetorhcological fluids (ferrofluids)
   - Stabilization of inorganic particles (TiO2, etc.) in paints
Many other such applications will occur to one skilled in the art, in view of the present disclosure.

## Claims

1. A peptidomimetic polymer comprising a coupled polypeptide moiety and a polypeptoid moiety, the polypeptide moiety consisting of a 5-mer peptide mimic of Mefp-5 comprising alternating residues of L-3, 4-dihydroxyphenylalanine (DOPA) and lysine, wherein the polypeptoid moiety comprises a poly-N-substituted glycine oligomer, wherein the N- substituent is methoxy ethyl.

2. A coating comprising a peptidomimetic polymer according to claim 1.

3. A coated metal work piece comprising a metal oxide surface having adhered thereto a polypeptidomimetic coating of claim 2.

4. A work piece according to claim 3 wherein the metal oxide surface is a working surface of a medical device.

5. A work piece according to claim 4 wherein the metal oxide surface comprises titanium oxide.

6. A coating as claimed in claim 2 which is an antifouling resistant coating.

## Patentansprüche

1. Peptidomimetisches Polymer, umfassend eine gekoppelte Polypeptid-Komponente und eine Polypeptoid-Komponente, wobei die Polypeptid-Komponente in einem 5-mer Peptid-Nachahmer von Mefp-5, umfassend sich abwechselnde Reste von L-3,4-Dihydroxyphenylalanin (DOPA) und Lysin, besteht, wobei die Polypeptoid-Komponente ein Poly-N-substituiertes Glycin-Oligomer, worin der N-Substituent Methoxyethyl ist, umfasst.

2. Beschichtung, umfassend ein peptidomimetisches Polymer gemäß Anspruch 1.

3. Beschichtetes Metallwerkstück, umfassend eine Metalloxid-Oberfläche, an welcher eine polypeptidomimetische Beschichtung gemäß Anspruch 2 haftet.

4. Werkstück gemäß Anspruch 3, wobei die Metalloxid-Oberfläche eine Arbeitsoberfläche einer medizinischen Vorrichtung ist.

5. Werkstück gemäß Anspruch 4, wobei die Metalloxid-Oberfläche Titanoxid umfasst.

6. Beschichtung, wie in Anspruch 2 beansprucht, welche eine anwuchsverhindernde (Antifouling) Beschichtung ist.

## Revendications

1. Polymère peptidomimétique comprenant un fragment polypeptide couplé et un fragment poly(peptoïde), le fragment polypeptide étant constitué d'un mimétique peptidique 5-mère de Mefp-5 comprenant des résidus alternés de L-3, 4-dihydroxyphénylalanine (DOPA) et de lysine, le fragment poly(peptoïde) comprenant un oligomère de glycine poly-N-substitué, le substituant N étant le méthoxy-éthyle.

2. Revêtement comprenant un polymère peptidomimétique selon la revendication 1.

3. Pièce de travail métallique revêtue comprenant une surface d'oxyde métallique comportant en adhésion dessus un revêtement polypeptidomimétique selon la revendication 2.

4. Pièce de travail selon la revendication 3, dans laquelle la surface d'oxyde métallique est une surface de travail d'un dispositif médical.

5. Pièce de travail selon la revendication 4, dans laquelle la surface en oxyde métallique comprend de l'oxyde de titane.

6. Revêtement selon la revendication 2, qui est un revêtement antisalissure.
